Europäisches Patentamt

European Patent Office

Office européen des brevets

(11) Publication number: **0 150 697**
A1

# EUROPEAN PATENT APPLICATION

(21) Application number: **84850385.0**

(22) Date of filing: **12.12.84**

(51) Int. Cl.⁴: **G 07 C 9/00,** G 07 C 11/00, G 06 K 9/00, A 61 B 5/10

(30) Priority: **29.12.83 SE 8307211**

(43) Date of publication of application: **07.08.85**
**Bulletin 85/32**

(84) Designated Contracting States: **DE FR GB IT**

(71) Applicant: **TELEFONAKTIEBOLAGET L M ERICSSON, S-126 25 Stockholm (SE)**

(72) Inventor: **Anderson, Knut Peter, Heleneborgsgatan 7 4trp, S-117 31 Stockholm (SE)**
Inventor: **Baltscheffsky, Patrik Gustav Vilhelm, Erikdahlbergsgatan 23, S-115 32 Stockholm (SE)**
Inventor: **Bengtsson, Anders Bengt, Senapsvägen 24, S-178 00 Ekerö (SE)**

(74) Representative: *Szemere, Fredrik et al,* **Telefonaktiebolaget L M Ericsson Patent Department, S-126 25 Stockholm (SE)**

(54) **Apparatus for verifying a person's identity by means of characteristic features of his hand.**

(57) Apparatus for verifying a person's identity with the aid of characteristic features of that person's hand. A data processing device compares measured characteristic features with previously registered, corresponding information concerning the same person, and video camera equipment registers the hand image in digital form in a memory. A light box is arranged to give the hand sufficient space so that it does not need to come into contact with the walls of the box. A pulsed light source sends light pulses which are short in relation to the image frequency, for preventing blurring due to movement in the information recorded by the video equipment.

# APPARATUS FOR VERIFYING A PERSON'S IDENTITY WITH THE AID OF CHARACTERISTIC FEATURES OF THAT PERSONS'S HAND

## TECHNICAL FIELD

**TITLE MODIFIED**
see front page

The invention relates to an apparatus for verifying a person's identity with the aid of characteristic features of that person's hand, without the hand needing to touch any part of the apparatus.

## BACKGROUND ART

It is already known to verify the identity of a person, e.g. for checking authorization to pass barrier, by recognizing certain characteristic features of the hand, e.g. fingerprints, lines on the palm, hand contour etc. A method of recognizing a person with guidance from certain characteristic attributes of the hand contour are discussed in a study with the title: "Automatic Palmprint Verification Study" by James R. Young and Robert W. Hammon, Rome Air Development Centre, Air Force Systems Command, Griffis Air Force Base, New York, 13441. All the known methods of the above-mentioned type are burdened with the drawback that the person's hand must come into contact with a surface, which can cause an unpleasant sensation, due to the surface being greasy, sticky or dirty.

## DISCLOSURE OF INVENTION

The object of the present invention is to enable verification of a person's identity based on the contour lines of the hand, without the latter needing to come into contact with any surface, the hand being kept freely in a generously dimensioned space, which furthermore affords a full view from the outside. This is achieved in accordance with the invention by the hand being illuminated with light rays, which give the same image, irrespective of the position of the hand, simultaneously as the consequences of keeping the hand free in the air, causing a blurred image, are eliminated. The invention is characterized by the disclosures in the claim.

## BRIEF DESCRIPTION OF DRAWING

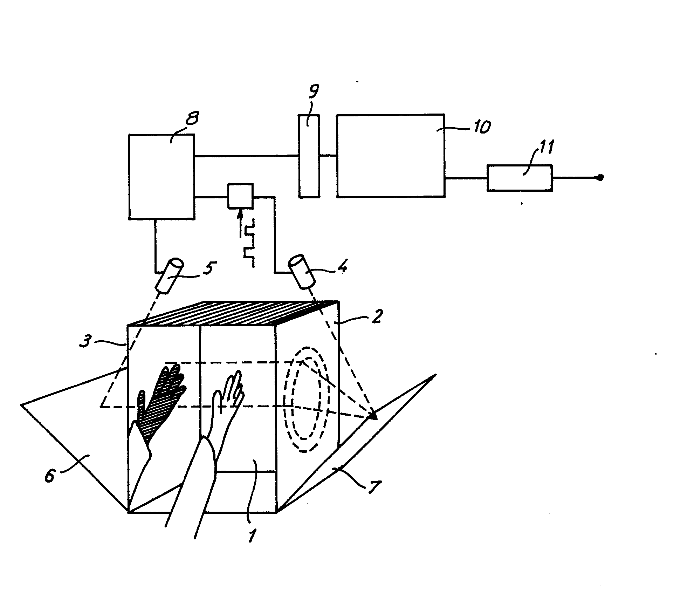

The invention will be described in detail below with the aid of an embodiment, and with reference to the accompanying drawing, schematically illustrating an inventive apparatus.

## BEST MODE FOR CARRYING OUT THE INVENTION

On the drawing there is illustrated a light box 1, having three walls, its front side being open to allow the insertion of a hand. The inner dimensions of the box are selected such that the hand does not need to come into contact with the walls, since the wall spacing horizontally is a few times the thickness of hand, and vertically a few times greater than the width of the hand. Two opposing walls 2,3 are made up from Fresnel lenses. The wall 2 is illuminated by a dot-like light source and forms a parallel light beam passing through the interior of the box 1 to the Fresnel lens 3 situated opposite. This focuses the parallel light rays for entry to a video camera 5. To limit the dimensions of the apparatus horizontally, two mirrors 6 and 7 are arranged such that their sloping positions deflect the paths of the incoming and outgoing light rays, respectively. The image sensed by the video camera is converted to binary signals by video signal equipment 8 in known manner, so that the hand image may be stored in binary form in a data memory 9.

Information concerning the hand and the identity of the person to whom it belongs must be stored in a data processor 10 before the hand is inserted in the box, in order to determine that a person's hand has the attributes that have been registered in the processor as characteristic for the person in question. Such data may be obtained from an identity card fed to the apparatus, the card having been programmed earlier, at a time when a number of measurements were taken and the mean values thereof registered together with the person's identity.

The calculation of hand features and the comparison with previously registered data belonging to a person are made according to the principles described in the Study mentioned above. According to the Study, there are measured a number of parameters, such as 30-40, pertaining to the hand, e.g. its perimeter, finger length etc. The values of these parameters are stored and utilized for comparison with corresponding values of a hand that has just been inserted in the apparatus. The values are compared individually and the deviations weighted and added. The conformity requirement may be fixed as desired. The described method is known per se from the Study, and does not constitute a subject of the invention.

With the aid of a program, the dataprocessor 10 compares the characteristic features of the image, registered in binary form, with the previously registered features. If the processor finds that the features of the image conform to a given degree with the previously stored informatioin, a signal is sent via I/O equipment 11, to later form the basis of a decision. Since substantially parallel light rays are used, the hand does not need to be placed on a plate, as in the already known devices, and it may be kept in different positions without this essentially affecting the measured values or the comparison result between the measured and the previously registered characteristic hand values to any degree.

Since the hand is held practically freely in the light box the measured values would become uncertain, if the hand were to flutter or shake. For this reason the light source 4 is pulsed with pulses synchronous with the image frequency, but which are considerably shorter. Together with the illumination by parallel rays, this enables the measurement of characteristic hand data without the hand needing to come into contact with any part of the apparatus.

The start of measurement occurs automatically when the entire hand has been projected onto the wall 3. This is effected by the position of the hand being calculated, and only when the entire hand has been postioned on the image field is the registration of the hand data in the memory started, so that the calculation and comparison operation can be initiated. The calculation results in a signal indicating whether the person in question is the one he represents himself to be.

The light rays in the embodiment are parallel, but some divergence is also possible. Furthermore, instead of the Fresnel lenses, an ordinary lens system may be used, which generates parallel or nearly parallel rays. The apparatus may also be supplemented with measuring devices known per se, for measuring the distance and angle of the hand with respect to a reference position, the devices sending corresponding correction signals to the computer 10.

## CLAIM

Apparatus for verifying a person's identity with the aid of characteristic features of that person's hand, including a calculation device which compares characteristic hand features with previously registered information concerning the same person, a light source projecting the hand contour onto an image pattern sensing device, and an image pattern registration device which stores the projected image in digital form in a memory, characterized in that the apparatus includes a sensing chamber sufficiently large for the hand not to need to come into contact with any part of the confines of the chamber, and in that the apparatus is provided with a collimator giving a light beam filling the active part of the chamber, as well as a device controlling the image information registration time such that it is considerably shorter than the time during which the hand can move a distance causing a blurred image.

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 206 441 (KONDO)<br><br>*Column 2, lines 39-63; figure 1* | 1 | G 07 C 9/00<br>G 07 C 11/00<br>G 06 K 9/00<br>A 61 B 5/10 |
| Y | US-A-4 236 082 (BUTLER)<br><br>*Column 3, line 46 – column 4, line 49; claims 1,4,5,; figure 1* | 1 | |
| A | US-A-4 186 378 (MOULTON)<br><br>*Column 1, line 40 – column 2, line 39; column 2, line 64 – column 5, line 49; figure 1*<br>& FR-A-2 398 351 | 1 | |
| D,A | J.R. Young, R.W. Hammon, "Automatic Palmprint Verification Study", Report No. RADC-TR-81-161, June 1981, Rome Air Development Center, Air Force Systems Command, Griffiss Air Force Base, New York 13441<br><br>*Pages 3-5, 9-12* | 1 | **TECHNICAL FIELDS SEARCHED (Int. Cl.4)**<br><br>G 07 C<br>G 06 K<br>B 07 C<br>A 61 B |

The present search report has been drawn up for all claims

| Place of search | Date of completion of the search | Examiner |
|---|---|---|
| STOCKHOLM | 28-03-1985 | BANDELIN H |